# EUROPEAN PATENT SPECIFICATION

(11) **EP 1 675 855 B1**
(45) Date of publication and mention of the grant of the patent: **10.12.2008**
(21) Application number: 04791711.7
(22) Date of filing: 18.10.2004
(51) Int. Cl.: C07D 471/04, C07F 1/12, C07F 15/00, C07F 9/60, A61K 31/4709, A61P 33/06

(54) **4-AMINO-QUINOLINE OUINOLIZIDINYL-AND QUINOLIZIDINYLALKYL-DERIVATIVES WITH ANTIMALARIAL ACTIVITY**
4-AMINO-CHINOLIN-, -CHINOLIZIDINYL- UND -CHINOLIZIDINYLALKYLDERIVATE MIT ANTIMALARIAWIRKUNG
DERIVES DE QUINOLIZIDINYLE DE 4-AMINO-QUINOLINE ET D'ALKYLE DE QUINOLIZIDINYLE A ACTIVITE ANTIPALUDEENNE

(30) Priority: 21.10.2003 IT MI20032044
(43) Date of publication of application: 05.07.2006
(73) Proprietor: Need Pharmaceuticals S.r.l., 20123 Milano (IT)
(72) Inventor: SPARATORE, Anna, Universita' Degli Studi Di Milano, 20122 Milano (IT); TARAMELLI, D., Universita' Degli Studi di Milano, 20122 Milano (IT); BASILICO, N., Universita' Degli Studi di Milano, 20122 Milano (IT); PARAPINI, S., Universita' Degli Studi di Milano, 20122 Milano (IT); SPARATORE, F., Universita' Degli Studi di Milano, 20122 Milano (IT); BOIDO, Vito, c/o Universita' Degli Studi di Milano, 20122 Milano (IT); CANU BOIDO, C., Universita' Degli Studi di Milano, 20122 Milano (IT)
(74) Representative: Trupiano, Federica
(86) International application number: PCT/IB2004/003393
(87) International publication number: WO 2005/037833

(56) References cited:
- BOIDO CANU ET AL.: "Preparazione e attività antileucemica di chinolizidinil-alchil derivati della 4-amminochinolina e della 9-amminoacridina" BOLLETTINO CHIMICO FARMACEUTICO, vol. 128, 1989, pages 212-215, XP009043694 cited in the application
- SPARATORE ET AL.: "Quinolizidine derivatives with antimicrobial activity" FARMACO, EDIZIONE SCIENTIFICA, vol. 42, no. 3, 1987, pages 159-174, XP009043709
- CHAUHAN P M S ET AL: "ANTIPARASITIC AGENTS: PART 5-SYNTHESIS OF 4-(SUBSTITUTED ARYL)AMINO-7-CHLOROQUINOLINES AS POTENTIAL ANTIMALARIAL AGENTS" INDIAN JOURNAL OF CHEMISTRY, JODHPUR, IN, vol. 25B, November 1986 (1986-11), pages 1142-1145, XP009037752
- BOIDO ET AL.: "Synthesis of quinolizidiyl derivatives as possible antineoplastic agents" STUDI SASSARESI, SEZIONE 2, ARCHIVIO BIMESTRALE DI SCIENZE MEDICHE E NATURALI, vol. 57, no. 5-6, 1979, pages 811-816, XP001205179 cited in the application

## Description

### Field of the invention

The present invention relates to antimalarial compounds, in particular to 4-amino-quinoline derivatives.

### Background of the invention

Malaria is an infectious disease caused by protozoa of the Genus *Plasmodium*. In humans, it is caused by four different parasite species: *Plasmodium vivax, P. malariae, P. ovale* and *P. falciparum,* the most virulent and the only lethal one. Malaria is considered, together with tubercolosis and AIDS, one of the most serious and diffused pathologies in the world.

Nowadays, few antimalarial drugs are available due both to worldwide diffusion of drug-resistant strains of *P. falciparum* and to the scarce number of compounds developed in the last 25 years (only 4 antimalarials out of about 1400 registered drugs in the world) (Olliaro P & Taylor WRJ, J. Exp. Biol., 206, 3753, 2003).

The resistance of *P. falciparum* to quinoline-type drugs, such as chloroquine, and antifolates, such as sulfadoxine/pyrimethamine, is ubiquitous and a number of multi-drug resistant strains have developed. There is therefore the urgent need for antimalarial drugs that are effective, cheap and easy to use in endemic areas, such as sub-Saharian Africa.

Strategies aimed at reaching this goal include: 1) optimisation of combined therapy with the available anti-malarial drugs in association with artemisine-derivatives (the only still active medicaments towards which resistance is not observed); 2) development of analogues of known compounds; 3) identification of new synthetic or natural substances (Rosenthal PJ, J. Exp. Biol. 2003, 206, 3735).

Identification and study of compounds belonging to the class of 4-aminoquinoline, such as chloroquine, [7-chloro-4-(4-diethylamino-1-methylbutylamino)quinoline], is a still valid approach, due to their efficacy, low toxicity and low cost. However, there is the need for chloroquine analogues which are also effective towards chloroquine-resistant strains of *P. falciparum*.

Among the 4-aminoquinoline chloroquine analogues synthesised so far, there are some derivatives whose dialkylaminoalkylamino chain at the 4-position of the quinoline nucleus is modified. In particular, Studi Sassaresi, 57 (1979) pp. 811-816 and Bollettino Chimico Farmaceutico (June 1989, vol. 128, pp. 212-215) report the following compounds: 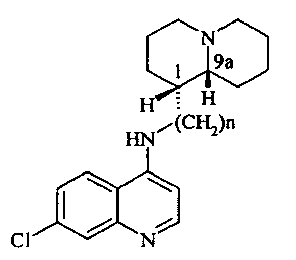 wherein, n is 1, 2 o 3,
in the form of pure enantiomers, in which the hydrogen atoms at the 1- and 9a- positions of the quinolizidinyl nucleous are in the *cis* conformation.

Despite the authors' hypotheses, the claimed antimalarial activity of these compounds has not been demonstrated.

### Detailed description of the invention

It has now been found that 4-amino-quinoline quinolizidinyl- and ω-(quinolizidinyl-1-yl)alkyl-derivatives have antimalarial activity and are active also towards chloroquine-resistant *P. falciparum* strains.

The present invention relates to the use of compounds of the general formula (I) 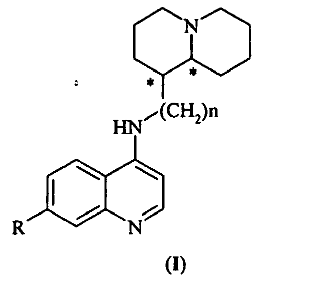 wherein:
R is selected from chlorine, bromine and trifluoromethyl; and
n is an integer from 0 to 3;
and pharmaceutically acceptable salts thereof for the preparation of medicaments for malaria therapy.
A further object of the invention are the compounds of the general formula (**I**) 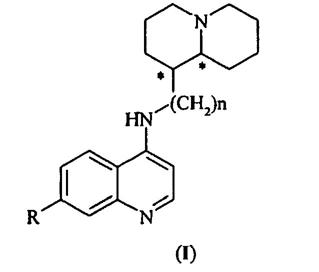
in which:
R is selected from chlorine, bromine and trifluoromethyl; and
n is an integer from 0 to 3;
with the proviso that, when R is chlorine and n is 1, 2 or 3, the hydrogen atoms at the 1- and 9a- positions of the quinolizidinyl nucleous are in the *trans* conformation;
and pharmaceutically acceptable salts thereof.

Unless otherwise indicated, the term "compounds of formula (**I**)" also comprises all the possible stereoisomers.

The term "pharmaceutically acceptable salts" comprises salts of the compounds of formula (**I**) with physiologically compatible acids, such as hydrochloric, phosphoric, sulfuric, tartaric and citric acid, as well as other acids commonly used in the pharmaceutical technique.

In particular, the following compounds are preferred, either as single enantiomers or in admixture with the corresponding optical antipodes:

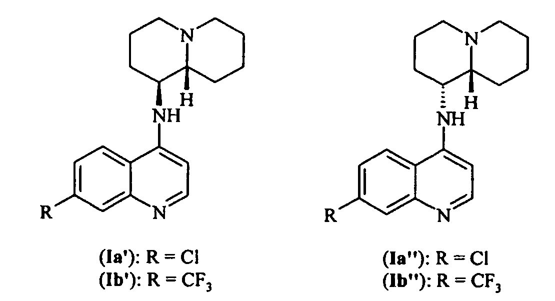

The invention also comprises the complexes of the general formula (**II**) 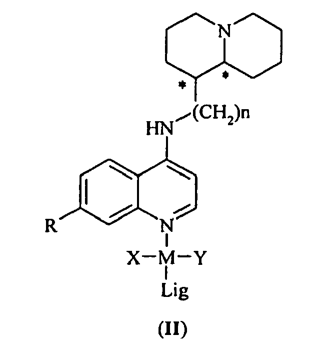 in which:
R is selected from chlorine, bromine, and trifluoromethyl;
n is an integer from 0 to 3;
M is a metal selected from gold, rhodium and ruthenium;
Lig is a ligand selected from:
   PR'₃, wherein R' is phenyl or C₂-C₄ alkyl, when M is gold; cyclooctadiene, when M is rhodium;
   another molecule of formula (**I**) when M is ruthenium;
X is PF₆⁻ when M is gold and Cl⁻ when M is rhodium or ruthenium;
Y is not present when M is gold or rhodium and is chlorine when M is ruthenium.

Particularly preferred is complex (**IIa**), wherein:
R is chlorine;
n is 1;
M is gold;
Lig is triphenylphosphine;
X is PF₆⁻;
and Y is not present.

The compounds of formula (**I**) wherein R is chlorine, n is an integer from 1 to 3 and the hydrogen atoms at the 1- and 9a- positions are in the *cis* conformation, are known and can be prepared by reaction between suitable ω-(quinolizidinyl-1α-yl)alkylamines and 4,7-dicloroquinoline, as described in Bollettino Chimico Farmaceutico, 128 (1989), pp. 212-215.

Analogously, compounds of formula (**I**) with the same steric configuration, in which R is bromine or trifluoromethyl and n is an integer from 1 to 3, can be prepared from the corresponding 7-substituted 4-chloro-quinolines.

The compounds of formula (**I**) in which R is chlorine, bromine or trifluoromethyl, n is an integer from 1 to 3 and the hydrogen atoms at the 1- and 9a- positions are in the *trans* configuration, can be prepared in the same way starting from the corresponding ω-(quinolizidinyl-1β-yl)alkylamines.

As for the quinolizidinylilalkylamines, (-)lupinylamine (n = 1) was prepared according to Sparatore, F. et al., Farmaco, Ed. Sci, 24 (1969) 587-621, whereas 2-(quinolizidinyl-1'α-yl)ethylamine (n=2) and 3-(quinolizidinyl-1'α-yl)propylamine (n=3) were prepared as described by Boido, V. et al., Farmaco, Ed. Sci., 34 (1979) 673-687. In all cases, *l*-lupinine (extracted from *Lupinus luteus* or *Lupinus hispanicus* seeds) was used as the starting material.

From racemic lupinine and *epi*-lupinine, obtained by synthesis (for example according to A. Sparatore et al., Farmaco, 44 (1989) 1193-1203) all the above mentioned compounds are obtained in the racemic form.

The preparation of epimeric quinolizidinylalkylamines is carried out in a similar way starting from d-*epi*-lupinine, which in turn can be obtained from *l*-lupinine by treatment with NaH in hot xylene (see, for example Iusco, G. et al., Farmaco, 51 (1996) 159-174).

The preparation of some quinolizidinylamines is illustrated in Scheme 1.

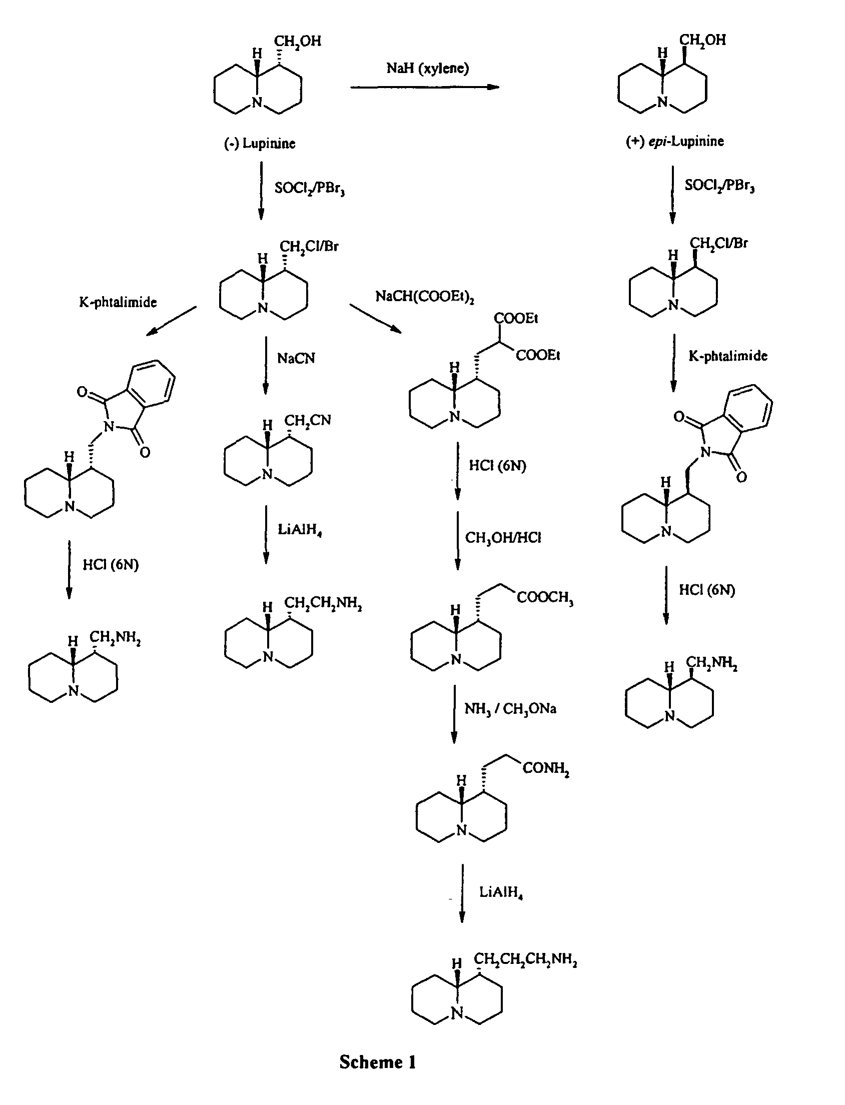

As for the compounds in which n is 0, 1-quinolizidinylamine was entirely synthesized, as shown in Scheme 2.

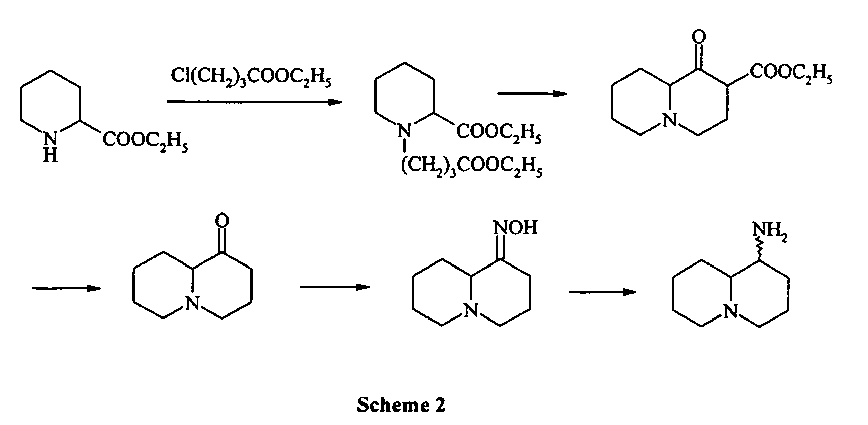

The pipecoline ester is alkylated with γ-chlorobutyric ester (Clemo, G.R., et al., J. Chem. Soc. (1931) 437-442 and W.A. Reckow, et. Al., J. Am. Chem. Soc. 74 (1952) 4960-4962), submitted to a Dieckmann condensation followed by decarboxylative hydrolysis, which leads to 1-quinolizidinone (nor-lupinone). The ketone is then converted to oxime, which is finally reduced to 1-quinolizidinylamine (Hadley, M.S. et al., J. Med. Chem. 28, (1985) 1843-1847). A mixture of diastereomers is obtained which, by reaction with an appropriate 7-substituted 4-chloro-quinoline, affords the corresponding compounds (**I**), always in the form of a diastereoisomeric mixture, which is subjected to chromatographic separation (Scheme 3).

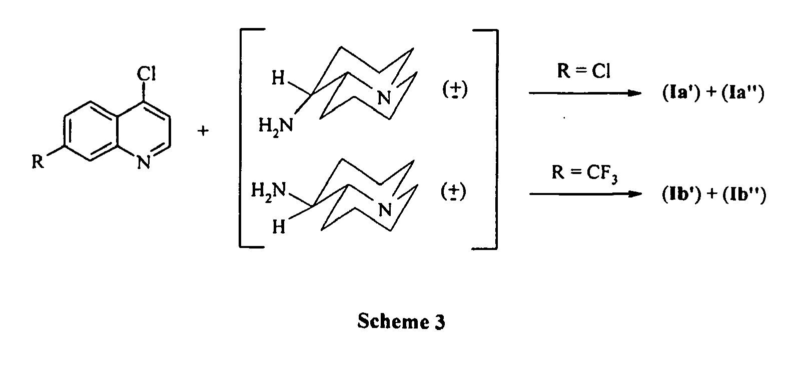

The complexes of formula (**II**) can be prepared according to the method described by Navarro, M. et al., J. Med. Chem. 40 (1997) 1937-1939 for the synthesis of the complex gold-chloroquine and Sanchez-Delgado, R. A. et al, J. Med. Chem. 39 (1996) 1095-1099 for the synthesis of the complexes rhodium-chloroquine and ruthenium-chloroquine.

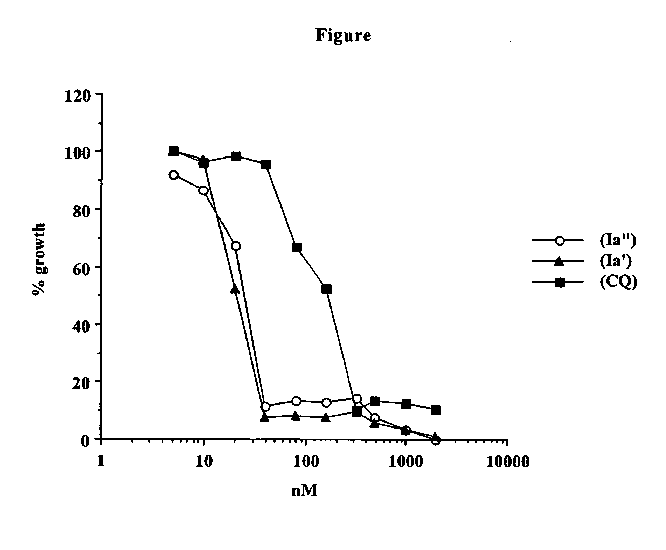

The compounds of the invention were tested *in vitro* on chloroquine-sensitive and chloroquine-resistant strains of *P. falciparum*. IC₅₀ values ranging from 15 to 30 nM were observed on chloroquine-sensitive *P. falciparum* strains; these values are comparable to those observed with chloroquine (chloroquine-sensitive IC₅₀= 27.8±9 nM). Surprisingly, IC₅₀ values ranging from 18 to 39 nM -significantly different from those of chloroquine (chloroquine-resistant IC₅₀= 150-300 nM)-, were also observed on chloroquine-resistant *P. falciparum* strains, with a 10-20 fold increase in activity compared to chloroquine, depending on the compounds and strains tested. As shown in the figure, compounds (**Ia'**) and (**Ia"**) proved 6-8 times more active than chloroquine on the W2 strain (chloroquine-resistant), with mean IC₅₀ values ranging from 10 to 25 nM. In particular, the figure shows the antimalarial effect of different doses of compounds (**Ia'**) and (**Ia"**) on the W2 *P. falciparum* strain, in comparison with the same doses of chloroquine (CQ). Parasites' growth was evaluated with a colorimetric assay for pLDH at 72 hours.

The compounds of the invention proved also effective *in vivo*, after intraperitoneal and oral administration in the experimental murine malaria model induced with *P. berghei*: the efficacy was comparable to that of chloroquine. Moreover, the toxicity of the compounds towards mammalian cells is low (IC₅₀=15.000-18.000 nM). Therefore, object of the present invention are also pharmaceutical compositions containing compounds of formula (I), salts or complexes thereof, in admixture with suitable excipients and/or carriers. The compositions will be suitable for administration through the oral or parenteral route and will be prepared, for example, according to the techniques and methods described in Remington's Pharmaceutical Sciences Handbook, XVII Ed. Mack Pub., N.Y., U.S.A.. The compounds of formula (**I**) and (**II**) will be contained at doses ranging from 1 to 1000 mg.

The invention will be hereinafter illustrated in more detail by means of some examples.

### EXAMPLES

### Example 1 - Synthesis of 4-(quinolizidinylalkyl)amino-quinoline

A mixture of 4,7-dichloro-quinoline or 7-bromo-4-chloroquinoline or 4-chloro-7-trifluoromethyl-quinoline (8 mmoles), quinolizidinylalkylamine (8 mmoles) and phenol (5 g) is heated for 4 hours at 180°C under magnetic stirring, using a reflux condenser with a KOH trap.

After cooling, the mixture is adjusted to strongly basic pH with NaOH and is extracted three times with ether. The ether solution is washed with 2N NaOH, then with water and finally extracted twice with 5% acetic acid. The acetic solution is then alkalized with 2N NH₃ and extracted with ether.

After evaporation of the solvent, a crystalline residue is obtained which is washed with some anhydrous ether and recrystallized from a dichloromethane-diethyl ether mixture or an ethanol-ether mixture.

The mother liquors are evaporated and the residue is purified by chromatography on a silica gel column eluting with a 98:2-93:7 CH₂Cl₂/MeOH gradient.

### Example 2 - Synthesis of 4-(quinolizidinyl)amino-7-chloroquinolines

A mixture of 4,7-dichloroquinoline (9.44 mmoles), 1-quinolizidinylamine (9.44 mmoles, diastereomeric mixture) and 6 g of phenol is heated for 4 hours at 180°C under magnetic stirring, using a reflux condenser with a KOH trap.

After cooling, the mixture is adjusted to strongly basic pH with NaOH and extracted four times with ether. The ether solution is washed with 2N NaOH, then with water and finally extracted twice with 5% acetic acid. The acetic solution is then alkalized with 2N NH₃ and extracted with ether.

After evaporation of the solvent a solid residue is obtained, which is chromatographed on silica gel eluting with a 99:1-92:8 CH₂Cl₂/MeOH gradient.

By evaporation of the fractions eluted with CH₂Cl₂/1-2% CH₃OH and washing of the residue with ether, a compound with a melting point of 168-169.2°C (**Ia"**) is obtained, whereas by evaporation of the fractions eluted with CH₂Cl₂/3-8% MeOH and washing of the residue with ether a compound with a melting point of235-238°C (dec.) (**Ia'**) is obtained.

### Example 3 - Synthesis of compound (IIa)

200 mg (0.4 mmoles) of triphenylphosphine gold chloride dissolved under reflux in 20 ml of acetonitrile are added with 148.8 mg (0.8 mmoles) of potassium hexafluorophosphate (KPF₆) and heating is continued for 30 minutes.

266.7 mg (0.81 mmoles) of 4-[(quinolizidinylmethyl)amino]-7-chloroquinoline is added, the mixture is refluxed under nitrogen for 48 hours, then allowed to cool; the resulting precipitate is filtered off (172 mg).

The filtrate is concentrated, added with ethyl ether (a few drops) and stored in the refrigerator. The separated solid is filtered and washed with anhydrous ether /acetonitrile (1:3). The solution is concentrated again, treated with ether and stored again in the refrigerator.

This procedure is carried out several times, each one filtering off the precipitate. Finally, the solution is evaporated to dryness and the residue, i.e. compound of formula (IIa), is washed and dried. Yield: 200 mg.

## Claims

1. Compounds of the general formula (**I**) 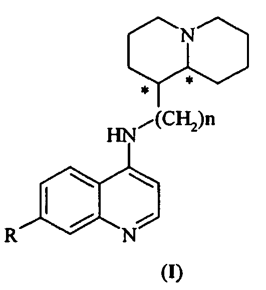 in which:
R is selected from chlorine, bromine and trifluoromethyl; and
n is an integer from 0 to 3;
with the proviso that, when R is chlorine and n is 1, 2 and 3, the hydrogen atoms at the 1- and 9a-positions of the quinolizidinyl nucleous are in the *trans* conformation;
and pharmaceutically acceptable salts thereof.

2. Compounds as claimed in claim 1 wherein R is selected from chlorine, bromine and trifluoromethyl and n is 0.

3. A compound as claimed in claim 2 selected from:

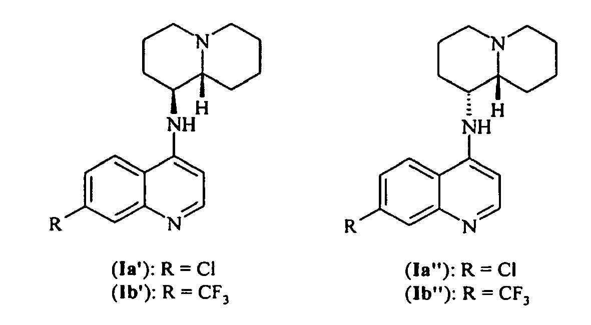

4. Complexes of the general formula (**II**) 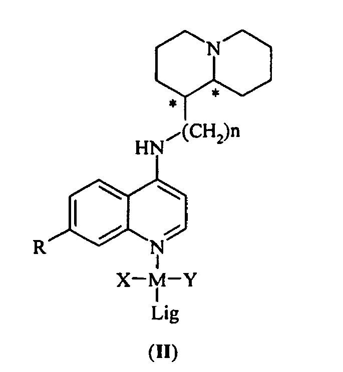 in which:
R is selected from chlorine, bromine and trifluoromethyl;
n is an integer from 0 to 3;
M is a metal selected from gold, rhodium and ruthenium;
Lig is a ligand selected from:
PR'₃, wherein R' is selected from phenyl or C₂-C₄ alkyl, when M is gold;
cyclooctadiene, when M is rhodium;
another molecule of formula (**I**) when M is ruthenium;
X is PF₆⁻ when M is gold and Cl⁻ when M is rhodium or ruthenium;
Y is not present when M is gold or rhodium and is chlorine when M is ruthenium,

5. Complex as claimed in claim 4 in which:
R is chlorine;
n is 1;
M is gold;
Lig is triphenylphosphine;
X is PF₆⁻;
Y is not present.

6. Use of compounds of formula (**I**): 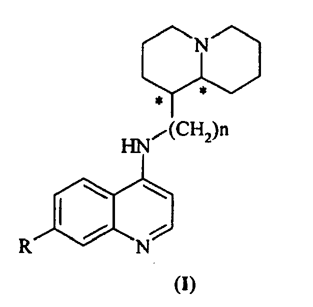 in which:
R is selected from chlorine, bromine; and
trifluoromethyl and n is an integer from 0 to 3; and
of the pharmaceutically acceptable salts thereof and of the complexes of claims 4 and 5 for the preparation of antimalarial medicaments.

7. The use as claimed in claim 6 in which the compound of formula (**I**) is selected from: 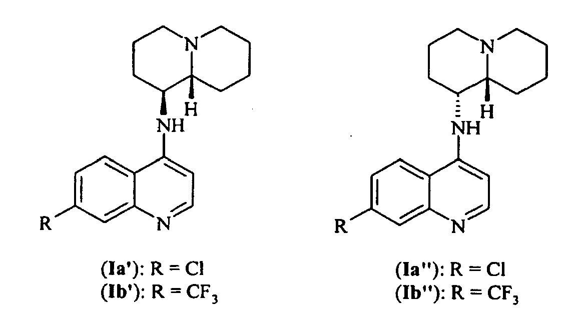 either as single enantiomers or in admixture with the corresponding optical antipodes.

8. Pharmaceutical compositions containing the compounds of formula (**I**) as defined in claim 6, the pharmaceutically acceptable salts thereof or the complexes of claims 4 or 5 in admixture with suitable excipients and/or carriers.

## Patentansprüche

1. Verbindungen der allgemeinen Formel (**I**) 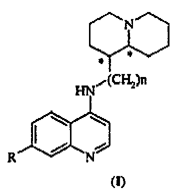 wobei:
R ausgewählt wird unter Chlor, Brom und Trifluormethyl; und
n eine ganze Zahl von 0 bis 3 ist;
mit der Bedingung, dass, wenn R Chlor ist und n 1, 2 und 3 ist, sich die Wasserstoffatome an den 1- und 9a-Positionen des Chinolizidinylkerns in der *trans*-Konformation befinden;
sowie pharmazeutisch zulässige Salze davon.

2. Verbindungen nach Anspruch 1, wobei R ausgewählt wird unter Chlor, Brom und Trifluormethyl, und n 0 ist.

3. Verbindung nach Anspruch 2, ausgewählt unter:

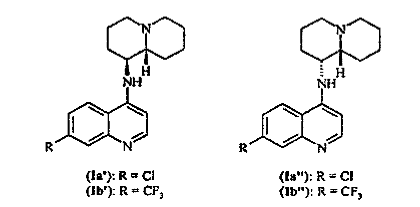

4. Komplexe der allgemeinen Formel (**II**) 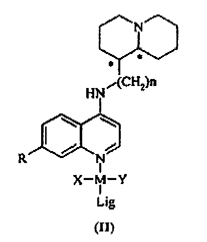 wobei:
R ausgewählt wird unter Chlor, Brom und Trifluormethyl;
n eine ganze Zahl von 0 bis 3 ist;
M ein Metall ist, ausgewählt unter Gold, Rhodium und Ruthenium;
Lig ein Ligand ist, ausgewählt unter:
PR'₃, wobei R' ausgewählt wird unter Phenyl oder C₂-C₄-Alkyl, wenn M Gold ist;
Cyclooctadien, wenn M Rhodium ist;
ein weiteres Molekül der Formel (**I**), wenn M Ruthenium ist;
X PF₆⁻ ist, wenn M Gold ist, und Cl⁻, wenn M Rhodium oder Ruthenium ist;
Y nicht vorhanden ist, wenn M Gold oder Rhodium ist, und Chlor ist, wenn M Ruthenium ist.

5. Komplex nach Anspruch 4, wobei
R Chlor ist;
n 1 ist;
M Gold ist;
Lig Triphenylphosphin ist;
X PF₆⁻ ist;
Y nicht vorhanden ist.

6. Verwendung von Verbindungen der Formel (I): 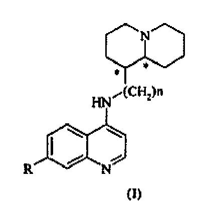 wobei:
R ausgewählt wird unter Chlor, Brom und Trifluormethyl; und
n eine ganze Zahl von 0 bis 3 ist; sowie
der pharmazeutisch zulässigen Salze davon und der Komplexe nach Anspruch 4 und 5 zur Herstellung von Antimalaria-Arzneien.

7. Verwendung nach Anspruch 6, wobei die Verbindung der Formel (I) ausgewählt wird unter: 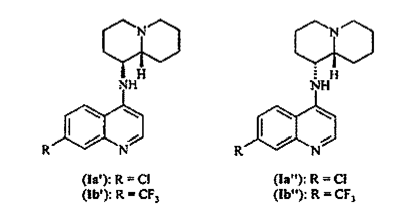 entweder als einzelne Enantiomere oder in Beimischung mit den entsprechenden optischen Antipoden.

8. Pharmazeutische Zusammensetzungen, enthaltend die Verbindungen der Formel (**I**), wie in Anspruch 6 definiert, die pharmazeutisch zulässigen Salze davon oder die Komplexe nach Anspruch 4 oder 5 in Beimischung mit geeigneten Hilfs- und/oder Trägerstoffen.

## Revendications

1. Composés de formule générale (I) 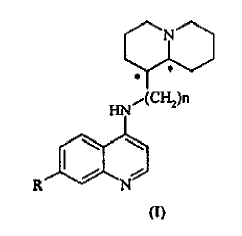 dans laquelle :
R est choisi parmi un atome de chlore, de brome et un groupe trifluorométhyle ; et
n est un nombre entier de 0 à 3 ;
à condition que si R est un atome de clore et si n vaut 1, 2 et 3, les atomes d'hydrogène en positions 1- et 9a du noyau de quinolizidinyle soient de conformation trans ;
et leurs sels pharmaceutiquement acceptables.

2. Composés selon la revendication 1, dans lesquels R est choisi parmi un atome de chlore, de brome et un groupe trifluorométhyle et n vaut 0.

3. Composé selon la revendication 2, choisi parmi :

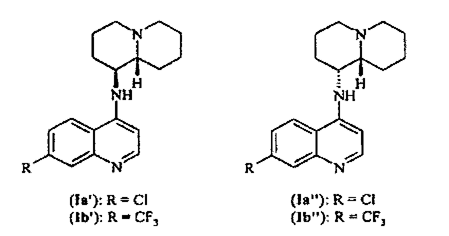

4. Complexes de formule générale (II) 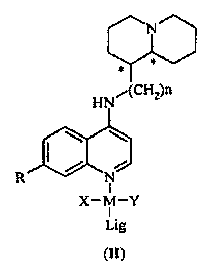 dans laquelle :
R est choisi parmi un atome de chlore, de brome et un groupe trifluorométhyle ;
n est un nombre entier de 0 à 3 ;
M est un métal choisi parmi l'or, le rhodium et le ruthénium ;
Lig est un ligand choisi parmi :
PR'₃, R' étant choisi parmi les groupes phényle ou alkyle en C₂ à C₄ si M est l'or ;
cyclo-octadiène, si M est le rhodium ;
une autre molécule de formule (I) si M est le ruthénium ;
X est PF₆⁻ si M est l'or et Cl⁻ si M est le rhodium ou le ruthénium ;
Y n'est pas présent si M est l'or ou le rhodium et est un atome de chlore si M est le ruthénium.

5. Complexe selon la revendication 4, dans lequel :
R est un atome de chloré ;
n vaut 1 ;
M est l'or ;
Lig est la triphénylphosphine ;
X est PF₆⁻ ;
Y n'est pas présent.

6. Utilisation des composés de formule (I) : 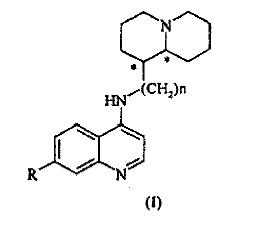 dans laquelle :
R est choisi parmi un atome de chlore, de brome ; et un groupe trifluorométhyle et n est un nombre entier de 0 à 3 ; et
de leurs sels pharmaceutiquement acceptables et des complexes des revendications 4 et 5 pour la préparation de médicaments anti-paludéens.

7. Utilisation selon la revendication 6, dans laquelle le composé de formule (I) est choisi parmi : 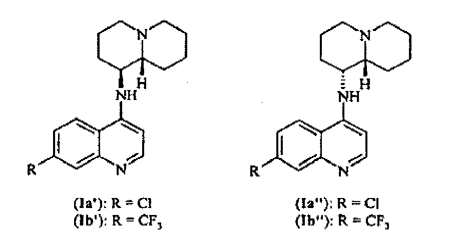 soit en tant qu'énantiomères simples soit en mélange avec les antipodes optiques correspondants.

8. Compositions pharmaceutique contenant les composés de formule (I) selon la revendication 6, leurs sels pharmaceutiquement acceptables ou les complexes des revendications 4 ou 5 en mélange avec des excipients et/ou véhicules appropriés.
